# EUROPEAN PATENT SPECIFICATION

(11) **EP 0 371 819 B1**
(45) Date of publication and mention of the grant of the patent: **12.07.1995**
(21) Application number: 89312519.5
(22) Date of filing: 30.11.1989
(51) Int. Cl.: B32B 7/06, B65D 65/40, B65D 77/20, B32B 31/00, B29C 65/00, A61M 35/00

(54) **Separable films**
Trennbare Filme
Films séparables

(30) Priority: 30.11.1988 JP 304624/88; 10.12.1988 JP 312361/88; 24.10.1989 JP 276581/89
(43) Date of publication of application: 06.06.1990
(73) Proprietor: NIHON TOKKYO KANRI COMPANY LIMITED, Tokyo (JP)
(72) Inventor: Inagaki, Hiromichi, Inuyama-shi Aichi-ken (JP)
(74) Representative: King, James Bertram

(56) References cited:
- EP-A- 0 208 395
- FR-A- 2 323 594
- US-A- 4 106 962

## Description

This invention relates to an easily separable laminated plastic film for packaging of foodstuffs and drugs.

Foodstuffs and drugs are customarily sealed in packs or containers for transportation and storage. Unless a part or the whole container is opened when heating up, cooking or steaming the entire container in an oven prior to consumption, the container is liable to be ruptured due to internal pressure.

Therefore, when heating up or cooking foodstuff, conventionally the foodstuff needs to be removed from the container on each occasion, or a part of the packing container needs to be opened by using a pair of scissors as example.

Further, with drugs such as surface acting drugs, paste drugs, saturated cloth drugs (poultices) or adhesive plaster they need to be attached to the skin by opening the packing container when used. In the case of these types of drugs, an adhesive agent has to be incorporated with the drugs for the purpose of providing adhesion, and the quantity of adhesive can be greater than the drug. This poses a problem especially to drug efficacy and life. In addition, if the quantity of drug is to be increased to provide drug efficacy and durability, the drug package is enlarged making outward appearance unsightly and increasing costs.

Such drugs may include a dessicant, insecticide, deoxydizer and as the individual packs are pervious require further packaging, for example in five or ten doses, into a larger external pack which is airtight. These small packs are then removed one by one from the large pack after opening. This type of packing system requires a double packing and once the external bag is opened, the residual small packs contact the air, whereby the drug efficacy and durability tend to be lost.

An object of this invention is to provide a laminated perforated film and manufacturing method that can be used with a packing machine as a single sheet of film when packing contents such as foodstuffs and the like and which after separation enables air and liquid to pass through the perforations of the film or allows a drug to contact the skin directly while providing easy separation and leaving a sheet of film with many perforations provided therein during usage.

Another object of this invention is to provide means for adjusting the separation strength without using a bonding adhesive agent between the aforementioned laminated/perforated film.

According to this invention there is provided a method for the manufacture of a laminated film of which the laminations comprise at least a perforated film and a closure film which are readily separable, which method is characterised by the steps of
a) bringing the closure film whilst in a softened deformable state into face-to-face contact with the perforated film,
b) applying pressure to the films to deform the closure film to enter the perforations to form a separable interlock, and
c) cooling the assembly.

This invention additionally provides a method for manufacturing an easily separable laminated perforated film combination, characterised by extruding a thin closure film from a fusion extruder and whilst molten and deformable pressing the film against one face of a perforated film to form a bond and thereafter cooling the assembly.

This invention provides also a readily separable laminated film assembly, characterised by a perforated film and a closure film wherein the closure film is detachably connected to the perforated film, the connection having been formed by deformation parts of the closure film into or around the perforations in the perforated film.

Further preferred features and packages incorporating the invention are defined in the sub-claims and hereinafter as examples.

This invention is further described and illustrated with reference to the accompanying drawings by way of example. In the drawings:-
- Figure 1: shows diagrammatically the manufacturing method for the laminated/perforated film of this invention,
- Figure 2: shows a cross-sectional view of the laminated/perforated film assembly for packings,
- Figure 3: shows a cross-sectional view with a liquid sealed therein using the film of this invention,
- Figure 4: shows a cross-sectional view with part of the laminated film separated and with the perforated inner film exposed,
- Figure 5: shows a cross-sectional view of an embodiment using the film according to this invention applied as the lid of a tray,
- Figure 6: shows a cross-sectional view with part of the laminated film in the lid of a tray separated,
- Figure 7: shows a cross-sectional view of an embodiment formed with small perforations in a crater profile,
- Figure 8: shows a cross-sectional view of the film assembly in a laminated state,
- Figure 9: shows diagrammatically perforations of crater profile,
- Figure 10: shows a laminated assembly in cross-section with perforations of crater profile,
- Figure 11: shows a cross-section of a packed percutaneous absorption drug using the film assembly of this invention,
- Figure 12: shows a cross-section of a paste percutaneous skin absorption drug, and
- Figure 13: shows a cross-section of an adhesive plaster in which the film relating to the present invention is used.

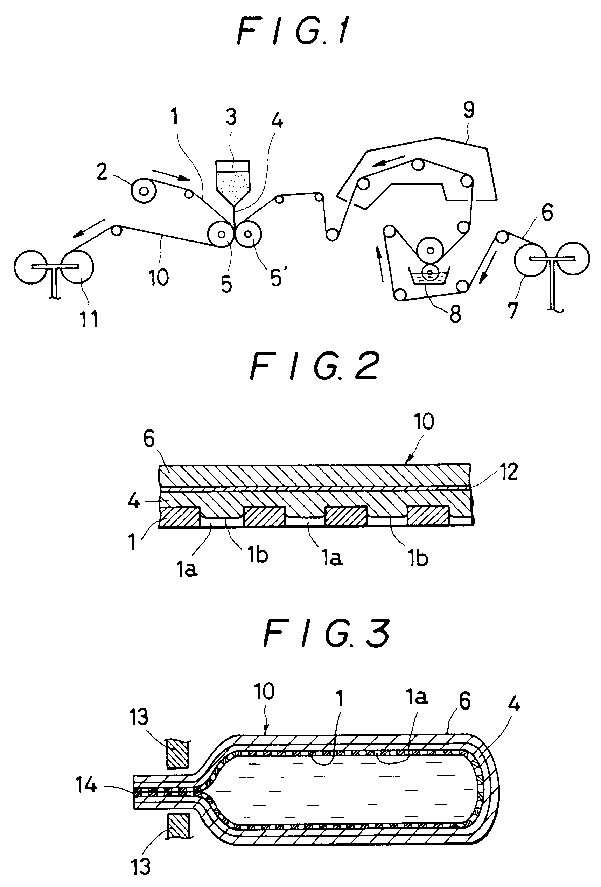

The method of manufacturing the laminated/perforated film relating to this invention is shown in Figure 1.

As shown, a perforated film 1 rolled around a roller 2 comprises a polypropylene film through which a number of 0.6mm diameter holes are perforated uniformly at 2mm pitch interval. A polyethylene or the like can be used as the basic material for this perforated film.

A fusion extruder 3 is provided and fused polyethylene 4 is extruded from this fusion extruder and is pressed against the aforesaid perforated film 1 in a molten fused state by rollers 5 and 5′ to provide a closure film layer.

Figure 2 shows in section the laminate after the fused polyethylene has been pressed on to form a sheet of laminated film. The film 4 in the fused state penetrates the perforations 1a of perforated film 1 when it is pressed between the rollers 5 and 5′. These depressed portions 1b uniformly enter all the perforations 1a of perforated film 2. However both parts are not fused together but there is an effective simulated bonding.

A polyester film 6 which is the base material film has been produced and rolled around a roller 7 enters between the rollers 5 and 5′ after passing through a bonding agent coating roller 8 and a drier 9, and is joined with the outside of film 4.

The packaging assembly 10 which has been manufactured in this way is rolled up in a rolling machine 11. The assembly has the bonding agent 12 provided by the coating roller 8 between the closure polyethylene film 4 and the polyester base film 6 and joining the two to each other.

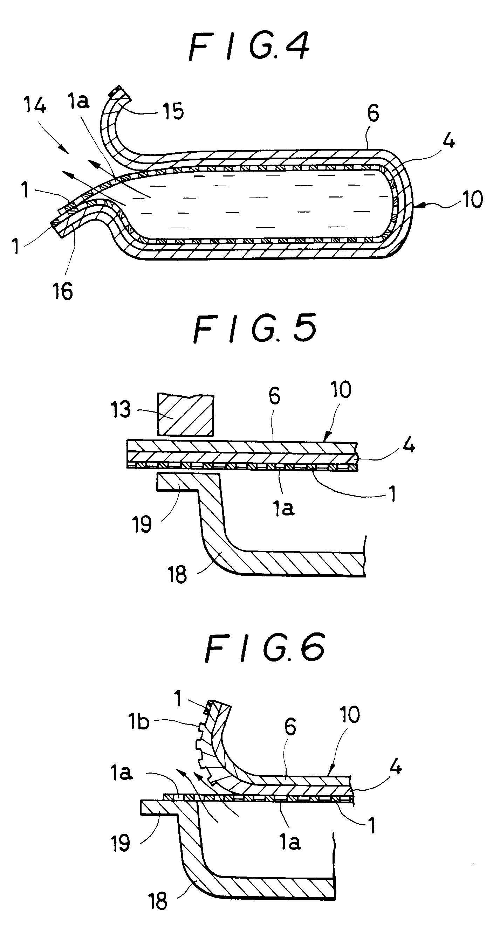

Figure 3 shows an assembly for sealed and packed sauce using the aforesaid laminated material with the pack 10 heat-sealed using a heat seal bar 13. Figure 4 shows the open condition when the closure and base films are peeled away together from each other to leave an open mouth 14. The perforated film 1 separates from the polyethylene closure film 4 at the front 15 which has a weaker strength because of the effective bonding. The perforated film 1 maintains the sealed state because of the heat-sealing but the sauce may flow out of the perforations of perforated film 1 when the container is squeezed or tipped. For this reason, because the sauce is not in a state for it to overflow immediately such liquid can be packed without problem. In the case of a foodstuff being heated electronically, the bag can be opened as described above for venting air through the perforation film 1.

Figure 5 and Figure 6 show an example using the package container 10 according to this invention as the lid of a container, where the container lid 10 is heat-sealed onto the rim 19 of a container 18 as illustrated in Figure 5. When the film is separated as shown in Figure 6, the perforated film 1 remains on the container 18 whilst the polyethylene closure film 4 and polyester base film 6 are separated and the perforated film 1 keeps the container 18 closed. Thus for foodstuff which needs to be separated from liquid the liquid can be drained easily through the perforations 1a while keeping the food within the container 18.

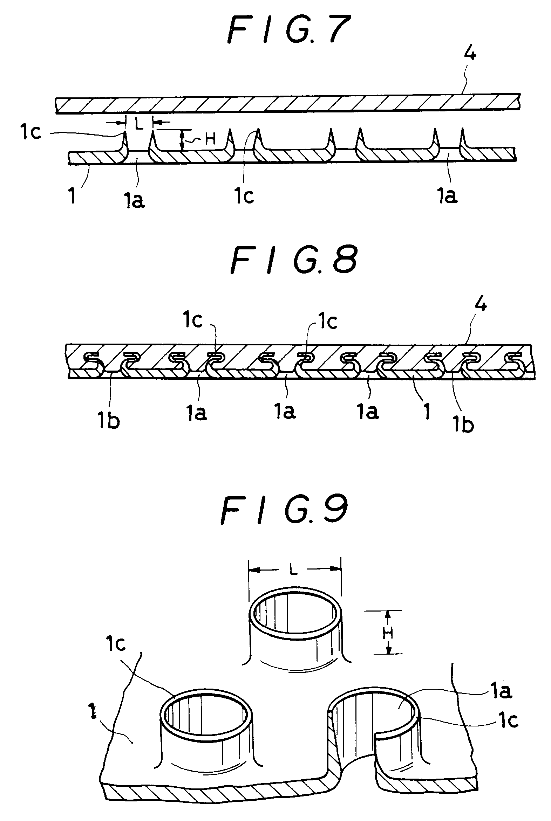

Figure 7 to Figure 10 show embodiments having increasing laminated strength and where the perforations 1a of film 1 presents crater-like profiles. With the fused closure film 4 pressed against the side of these profiles the rib 1c locks into the film 4, and the bond strength is increased.

For forming the perforated film 1 having perforations 1c of crater profiles, the polyethylene film is pressed against a roller having apertures for creating the perforations at the stage where this material, for example, a polyethylene film is still in the molten state. In addition the perforations can be created by a needle shape perforating tool operated from one face. Any of these methods are usable to produce the protruding rim 1c and the diameter can also be adjusted. The vacuum method is preferred as a means for achieving these conditions easily.

The height of rim 1c can be adjusted by the molten state of the film. For example, if the molten state of the film is high then the height of rim 1c becomes greater and if the molten state is low then the said height becomes lower. This change also depends on the nature of the plastic so needs to be known empirically for respective films. This changes depending on the environment, such as the room temperature, of the perforated film 1.

The quality of closure film 4 to be joined to the perforated film 1 is optional, but the molten state of film 4 when being pressed against the perforated plastic film 1 is such that the rim 1c on the perforated film enters the wall of film 4 and is deformed by the pressing action thus the state immediately after the formed film has been extruded is optimum.

The embodiments are explained in more detail hereinafter.

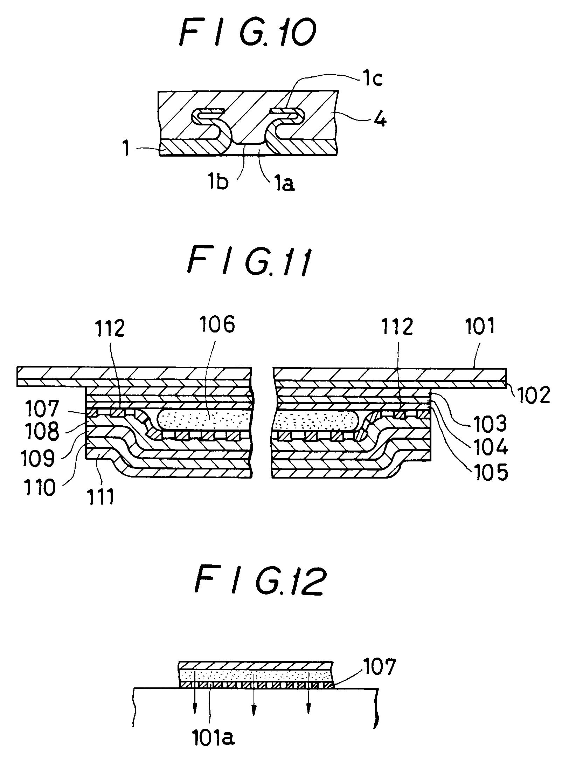

The perforated film 1, having perforations 1a equipped with the rim 1c which are formed in the polyethylene material by a vacuum process and the film 4, consisting of a polypropylene material when in the semi-molten or soft fused state immediately after the extrusion are brought together face to face and passed between the pressing rollers 5,5′ to force the rim 1c of the perforations 1a into the wall of film 4 and to collapse the rim by deformation. This state is illustrated in detail in Figure 10. Figure 9 shows the perforations 1a in the perforated plastic film, and the diameter L and height H of rim 1c are defined when forming by vacuum. For enhancing the bonding strength, it is sufficient to enlarge the diameter L and the height H and also to increase the number of perforations 1a. However, the strength can be increased by increasing H or L or the number of perforations with account being taken of the area for ventilation and penetration into the interior through the perforations.

Examples using the film of this invention for the packaging of a drug are shown in Figure 11 to Figure 13.

In Figure 11 an adhesive carrier 101 of a soft vinyl chloride, and an adhesive bonding layer 102 is to be adhered directly to a body surface. A nylon film 103 is secured to the centre of bonding agent layer 102 and an aluminium film 104 is adhered onto the surface of this nylon film, with a polyethylene film 105 secured onto this surface.

A drug 106 in paste form is retained by perforated film 107 with the perforations of 0.5mm diameter provided at 1mm pitch in a polyethylene film. A polypropylene film 108 is bonded to the outside of this perforated film 107.

The bonding is effected by forcing the film 108, when soft, into the perforations of film 107 by rollers. A covering polypropylene film 109 with an aluminium film 110 and a nylon film 111 completes the assembly with the polypropylene film 108 through to the nylon film 111 being a unitary laminated body. As the perforated film 107 is also bonded to the assembly with further processing such as bag making, heat-sealing, the assembly can be handed as one sheet of film.

A heat-sealed area 112 is provided for sealing in the drug 106, between the perforated film 107 and the film 105 which are fused to each other.

Figure 12 shows the use of the film attached to a body after the polypropylene film 108 is peeled away from the perforated film 107. The drug 106 is then covered only by the perforated film 107.

The drug 106 may ooze out through the perforations 101a of perforated film 107 and becomes effective on the body.

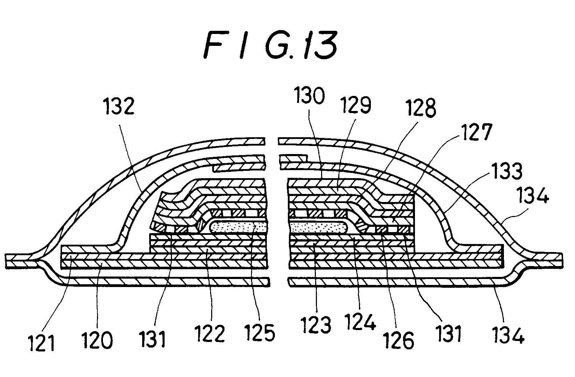

Figure 13 shows an embodiment being applied to an adhesive plaster coated with a drug, wherein a soft vinyl chloride film 120 with a bonding agent layer 121 on the surface is connected with a nylon film 122, an aluminium film 123, a polyethylene film 124 with a drug 125 encased in a perforated film 126 with a polypropylene film 127 bonded thereto in a releasable manner as hereinbefore described. An aluminium film 129, a nylon film 130 and a polypropylene film 128 all form a single laminated body.

The heat sealed portion 131 of polyethylene film 124 and perforated film 126 contains the drug 125. Covers 132 and 133 close the external side of the package provided with a protective closure 134.

In the case of an adhesive plaster of this construction the bonding of the perforated film 126 is easily peeled away after opening the exterior 134, breaking away the covers 132 and 133 and grasping the polypropylene film 128.

This assembly can be used as an adhesive plaster by securing the adhesive bonding layer 121 onto a body similar to the preceding embodiment.

As referred to above, the present invention has the pressed fused film forced onto and in the perforated film to cause the fused film to enter the perforations thereby achieving a releasable bonding.

As a result productivity is enhanced and the external appearance similar to a sheet of film which can be handled as such for making a package or a lid for a container. Consequently, double processing required conventionally for the interior and the exterior parts become unnecessary.

The releasable bonding strength can be selected by the formation of the rims of the perforations in the perforated film and adjusting the height and number.

Moreover, by applying the film according to this invention to such chemicals as paste-form drugs, efficacy can be improved and durability can be lengthened.

## Claims

1. A method for the manufacture of a laminated film of which the laminations comprise at least a perforated film and a closure film which are readily separable, which method is characterised by the steps of
a) bringing the closure film whilst in a softened deformable state into face-to-face contact with the perforated film,
b) applying pressure to the films to deform the closure film to enter the perforations to form a separable interlock, and
c) cooling the assembly.

2. A method for manufacturing an easily separable laminated perforated film combination, characterised by extruding a thin closure film from a fusion extruder and whilst molten and deformable pressing the film against one face of a perforated film to form a bond and thereafter cooling the assembly.

3. A method as claimed in Claim 1 or 2, characterised by passing the perforated film and extruded closure films between pressure rollers and simultaneously bonding to the closure film a base film.

4. A readily separable laminated film assembly, characterised by a perforated film (1) and a closure film (4) wherein the closure film (4) is detachably connected to the perforated film (1), the connection having been formed by deformation of parts (1b) of closure film (4) into or around the perforations (1a) in the perforated film.

5. A container formed from a laminated film assembly characterised in that the assembly (10) includes an impervious closure film (4) separably connected with a perforated film (1) capable of being heat-sealed, the closure film (4) having been extruded from a fusion extruder (3) and pressed against the perforated film (1) between rollers (5,5') whilst in the softened state, the container having been formed by heat sealing adjacent parts of the perforated film (1), the container being openable by separating the closure film (4) from the perforated film (1).

6. A method in accordance with any one of Claims 1 to 3 or a laminated film assembly in accordance with Claim 4 or a container in accordance with Claim 5, characterised in that perforated film (1) has perforations (1a) with rims (1c) which protrude in crater-like form into the closure film (4), the rims (1c) being pressed into the closure film (4) whilst same is in a softened state whereby the rims (1c) are deformed within the wall of the film (4).

7. A method in accordance with Claim 6, characterised in that the separation strength between the films (1) and (4) is selected by adjusting the height, diameter and number of the rims (1c) associated with perforations (1a).

8. A package containing a percutaneous absorption drug characterised in that the drug (106) is sealed within a laminated film assembly according to Claim 4 with the perforated film (107) innermost, the closure film comprising a composite laminate (108,109,110) separably bonded onto one face of the perforated film (107).

9. An adhesive plaster containing a drug characterised in that the drug (125) is sealed between a support film assembly (120 to 124) and a laminated film assembly (126,127) according to Claim 4, the support assembly having an adhesive coating (121) extending beyond the laminated film assembly (126,127) area containing the drug, the perforated film (126) being adjacent the drug (125) which may permeate through the perforations on separation of the closure film assembly (127 to 130).

## Patentansprüche

1. Ein Verfahren zur Herstellung eines geschichteten Films, bei dem die Schichten mindestens einen gelochten Film und einen Abdichtfilm umfassen, wobei die besagten Filme ohne weiteres voneinander trennbar sind, und zwar ist das Verfahren durch die Schritte gekennzeichnet, die darin bestehen,
a) daß der Abdichtfilm, während sich dieser in einem erweichten, verformbaren Zustand befindet, mit dem gelochten Film in flächenmäßigen Kontakt gebracht wird,
b) daß auf die Filme Druck ausgeübt wird, um den Abdichtfilm zu verformen, so daß dieser zwecks Erzielung einer trennbaren Einrastverbindung in die Lochungen eindringt, und
c) daß das zusammengesetzte Material gekühlt wird.

2. Ein Verfahren zur Herstellung eines ohne weiteres trennbaren, gelochten Film umfassenden geschichteten Verbundstoffs, gekennzeichnet durch das Extrudieren eines dünnen Abdichtfilms aus einem Schmelzextruder und das Anpressen des Films im geschmolzenen und verformbaren Zustand an eine Oberfläche eines gelochten Films, um eine Verbindung und danach Kühlung des zusammengesetzten Materials zu bewirken.

3. Ein Verfahren nach Anspruch 1 oder 2, dadurch gekennzeichnet, daß der gelochte Film und die extrudierten Abdichtfilme zwischen Druckwalzen hindurchgeleitet und gleichzeitig ein Basisfilm mit dem Abdichtfilm verbunden wird.

4. Ein ohne weiteres trennbares, aus geschichteten Filmen zusammengesetztes Material, gekennzeichnet durch einen gelochten Film (1) und einen Abdichtfilm (4), bei dem der Abdichtfilm (4) ablösbar mit dem gelochten Film (1) verbunden ist und die Verbindung durch Verformung von Teilen (1b) des Abdichtfilms (4) in oder um die Lochungen (1a) in dem gelochten Film bewirkt wurde.

5. Ein aus einem geschichtete Filme umfassenden zusammengesetzten Material gebildetes Behältnis, dadurch gekennzeichnet, daß das zusammengesetzte Material (10) einen undurchlässigen Abdichtfilm (4) umfaßt, der trennbar mit einem heißsiegelbaren gelochten Film (1) verbunden ist, wobei der Abdichtfilm (4) aus einem Schmelzextruder (3) extrudiert und im erweichten Zustand zwischen Walzen (5, 5') an den gelochten Film (1) gepreßt wurde, und das Behältnis durch Heißsiegeln anschließender Teile des gelochten Films (1) gebildet wurde und durch Trennen des Abdichtfilms (4) von dem gelochten Film (1) geöffnet werden kann.

6. Ein Verfahren nach einem der Ansprüche 1 bis 3 bzw. ein geschichte Filme umfassendes zusammengesetztes Material nach Anspruch 4 bzw. ein Behältnis nach Anspruch 5, dadurch gekennzeichnet, daß der gelochte Film (1) Lochungen (1a) mit Rändern (1c) aufweist, die in kraterartiger Form in den Abdichtfilm (4) vorstehen, wobei die Ränder (1c) in den Abdichtfilm (4) gepreßt werden, während dieser im erweichten Zustand ist, was zur Folge hat, daß die Ränder (1c) innerhalb der Wand des Filmes (4) verformt werden.

7. Ein Verfahren nach Anspruch 6, dadurch gekennzeichnet, daß der Trennwiderstand zwischen den Filmen (1) und (4) durch Einstellen der Höhe, des Durchmessers und der Anzahl der mit den Lochungen (1a) in Verbindung stehenden Ränder (1c) gewählt wird.

8. Eine eine Droge für Hautabsorption enthaltende Packung, dadurch gekennzeichnet, daß die Droge (106) innerhalb eines geschichtete Filme umfassenden zusammengesetzten Materials nach Anspruch 4 gesiegelt ist, wobei der gelochte Film (107) die innerste Schicht bildet und der Abdichtfilm einen trennbar mit einer Oberfläche des gelochten Films (107) verbundenen zusammengesetzten Schichtstoff (108, 109, 110) umfaßt.

9. Ein eine Droge enthaltendes Heftpflaster, dadurch gekennzeichnet, daß die Droge (125) zwischen einem zusammengesetzten Trägerfilmmaterial (120 bis 124) und einem geschichtete Filme umfassenden zusammengesetzten Material (126, 127) nach Anspruch 4 gesiegelt ist, wobei das zusammengesetzte Trägermaterial einen Klebstoffüberzug (121) umfaßt, der sich über den die Droge enthaltenden Bereich des geschichtete Filme umfassenden zusammengesetzten Materials (126, 127) hinaus erstreckt, wobei sich der gelochte Film (126) anschließend an der Droge (125) befindet, die bei Trennung des zusammengesetzten Abdichtfilmmaterials (127 bis 130) durch die Lochungen dringen kann.

## Revendications

1. Procédé de fabrication d'un film feuilleté dont les couches comprennent au moins un film perforé et un film de fermeture qui sont facilement séparables, ledit procédé étant caractérisé par les étapes de
a) mise en contact du film de fermeture à l'état déformable ramolli face à face avec le film perforé,
b) application de pression sur les films pour déformer le film de fermeture en le faisant pénétrer dans les perforations pour former un interverrouillage séparable, et
c) refroidissement de l'ensemble.

2. Procédé de fabrication d'une combinaison de films perforés feuilletés facilement séparables, caractérisé par l'extrusion d'un film de fermeture mince à partir d'une extrudeuse à fusion et, pendant que le film est fondu et déformable, la pression du film contre une face d'un film perforé pour former une liaison, puis le refroidissement de l'ensemble.

3. Procédé selon Revendication 1 ou 2, caractérisé par le passage du film perforé et des films de fermeture extrudés entre des rouleaux de pression et le collage simultané d'un film de base au film de fermeture.

4. Ensemble de films feuilletés facilement séparables caractérisé par un film perforé (1) et un film de fermeture (4), dans lequel le film de fermeture (4) est relié au film perforé (1) de manière amovible, la liaison ayant été formée par la déformation des parties (1b) du film de fermeture (4) dans les perforations (1a) du film perforé ou autour de celles-ci.

5. Récipient formé d'un ensemble de films feuilletés caractérisé en ce que l'ensemble (10) comprend un film de fermeture imperméable (4) relié de manière amovible à un film perforé (1) pouvant être scellé par chauffage, le film de fermeture (4) ayant été extrudé à partir d'une extrudeuse à fusion (3) et pressé contre le film perforé (1) entre des rouleaux (5, 5') pendant qu'il était à l'état ramolli, le récipient ayant été formé par scellage thermique des parties adjacentes du film perforé (1), le récipient pouvant être ouvert par séparation du film de fermeture (4) du film perforé (1).

6. Procédé selon l'une quelconque des Revendications 1 à 3 ou ensemble de films feuilletés selon la Revendication 4, ou récipient selon la Revendication 5, caractérisé en ce que le film perforé (1) a des perforations (1a) ayant des rebords (1c) qui font saillie en forme de cratère dans le film de fermeture (4), les rebords (1c) étant pressés dans le film de fermeture (4) pendant que ce dernier est à l'état ramolli, de telle sorte que les rebords (1c) sont déformés dans la paroi du film (4).

7. Procédé selon la Revendication 6, caractérisé en ce que la force de séparation entre les films (1) et (4) est choisie par ajustement de la hauteur, du diamètre et du nombre des rebords (1c) correspondant aux perforations (1a).

8. Emballage contenant une drogue à absorption percutanée caractérisé en ce que la drogue (106) est scellée à l'intérieur d'un ensemble de films feuilletés selon la Revendication 4, le film perforé (107) étant à l'intérieur, le film de fermeture comprenant un feuilleté composé (108, 109, 110) collé de manière amovible sur une face du film perforé (107).

9. Sparadrap adhésif contenant une drogue caractérisé en ce que la drogue (125) est scellée entre un ensemble de films de support (120 à 124) et un ensemble de films feuilletés (126, 127) selon la Revendication 4, l'ensemble de support ayant un revêtement adhésif (121) s'étendant au-delà de la surface de l'ensemble de films feuilletés (126, 127) contenant la drogue, le film perforé (126) étant adjacent à la drogue (125) qui peut passer par les perforations lors de la séparation de l'ensemble de films de fermeture (127 à 130).
